(19) Europäisches Patentamt
European Patent Office
Office européen des brevets

(11) Veröffentlichungsnummer: **0 206 074**
**B1**

(12) **EUROPÄISCHE PATENTSCHRIFT**

(45) Veröffentlichungstag der Patentschrift: **01.08.90**

(21) Anmeldenummer: **86107838.4**

(22) Anmeldetag: **09.06.86**

(51) Int. Cl.⁵: **C 07 C 275/18,**
C 07 C 273/18, C 07 C 271/10,
C 07 C 269/02, C 08 F 20/36,
C 08 F 22/22, A 61 K 6/02

# (54) (Meth)-acrylsäureester und ihre Verwendung.

(30) Priorität: **20.06.85 DE 3522005**

(43) Veröffentlichungstag der Anmeldung:
**30.12.86 Patentblatt 86/52**

(45) Bekanntmachung des Hinweises auf die Patenterteilung:
**01.08.90 Patentblatt 90/31**

(84) Benannte Vertragsstaaten:
**AT BE CH DE FR GB IT LI NL SE**

(56) Entgegenhaltungen:
**EP-A-0 023 685**
**EP-A-0 023 686**
**EP-A-0 171 847**
**EP-A-0 209 700**
**DE-A-2 816 823**

(73) Patentinhaber: **BAYER AG**
**D-5090 Leverkusen 1 Bayerwerk (DE)**

(72) Erfinder: **Winkel, Jens, Dr.**
**Hahnenweg 6**
**D-5000 Köln 80 (DE)**
Erfinder: **Bömer, Bruno, Dr.**
**Max-Planck-Strasse 53**
**D-5060 Bergisch-Gladbach 2 (DE)**
Erfinder: **Süling, Carlhans, Dr.**
**Carl-Leverkus-Strasse 10**
**D-5068 Odenthal (DE)**
Erfinder: **Reiners, Jürgen, Dr.**
**Carl-Rumpff-Strasse 57**
**D-5090 Leverkusen 1 (DE)**
Erfinder: **Podszun, Wolfgang, Dr.**
**Wolfskaul 4**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 206 074 B1

Courier Press, Leamington Spa, England.

**Beschreibung**

Die Erfindung betrifft neue Acrylsäure- und methacrylsäurederivate, im folgenden (Meth)-acrylsäurederivate genannt, von Tricyclodecanen und ihre Herstellung. Die neuen Verbindungen können als Monomere für die Anwendung in Dentalwerkstoffen eingesetzt werden.

Aus der DE—OS 2 931 926 sind Dentalmassen bekannt, die polymerisierbare (Methy)-acrylsäureester von Tricyclodecanen enthalten. Diese Dentalmassen zeigen bei der Anwendung jedoch immer noch einen nicht gewünschten Polymerisationsschrumpf.

Es wurden neue (Meth)-acrylsäure-Derivate von tricyclo-decanen der Formel

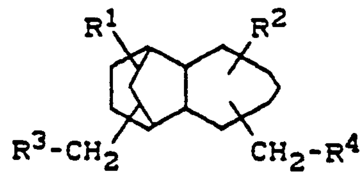

(I),

in der

$R^1$ und $R^2$ gleich oder verschieden sind und Wasserstoff, Niederalkyl, Niederalkoxy, Halogen oder Trifluoromethyl bedeuten, und

$R^3$ und $R^4$ gleich oder verschieden sind und für die Gruppen

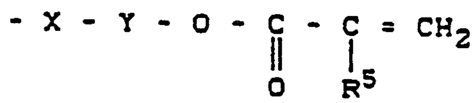

stehen, wobei

X ein zweibindiges Brückenglied aus der Gruppe

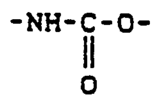

oder

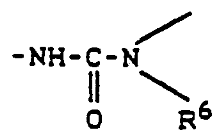

und

Y eine geradkettige oder verzweigte Kohlenwasserstoffkette mit 2 bis 10 Kohlenstoffatomen ist, die gegebenenfalls Saurestoffbrücken enthalten kann und durch gegebenenfalls 1 bis 4 Acrylat- oder Methacrylatrest substituiert ist, wobei

$R^5$ für Wasserstoff oder Methyl und

$R^6$ für Niederalkyl oder Phenyl stehen, gefunden.

Eine weitere Gruppe derartiger (Meth)-acrylsäure-Derivate wird in der parallelen EP—A—0 209 700 beschrieben.

Dentalmassen, bei denen von den erfindungsgemäßen (Meth)-acrylsäure-Derivaten von Tricyclodecanen ausgegangen wird, zeigen überraschenderweise einen wesentlich geringeren Polymerisationsschrumpf und sind daher für die Anwendung in der Praxis besonders geeignet.

Im Rahmen der vorliegenden Erfindung können die Substituenten die folgende Bedeutung haben:

Niederalkyl kann einen geradkettigen oder verzweigten Kohlenwasserstoffrest mit 1 bis etwa 6 Kohlenstoffatomen bedeuten. Beispielsweise seien die folgenden Niederalkylreste genannt: Methyl, Ethyl, Propyl, Isopropyl, Butyl, tert. Butyl, Isobuty, Pentyl, Isopentyl, Hexyl und Isohexyl. Bevorzugt werden der Methyl- und der Ethylrest.

Niederalkoxy kann ein über Sauerstoff gebundener geradkettiger oder verzweigter Kohlenwasserstoffrest mit 1 bis etwa 6 Kohlenstoffatomen bedeuten. Beispielsweise seien die folgenden Niederalkoxyreste genannt: Methoxy, Ethoxy, Propoxy, Isopropoxy, Butoxy, Isobutoxy, Pentoxy, Isopentoxy, Hexoxy und Isohexoxy. Bevorzugt sind der Methoxy- und der Ethoxyrest.

Halogen kann Fluor, Chlor, Brom oder Jod bedeuten. Bevorzugte Halogene sind Fluor und Chlor.

Y ist im allgemeinen eine geradkettige oder verzweigte Kohlenwasserstoffkette mit 2 bis 10 Kohlenstoffatomen, die durch gegebenenfalls 1 bis 4 Methacrylat- oder Acrylatreste substituiert ist. Die Kohlenwasserstoffkette kann gegebenenfalls 1 bis 3, bevorzugt 1 bis 2, Sauerstoffgruppen enthalten. Beispielsweise seien die folgenden Bedeutungen für Y genannt: Ethylen, Propylen, 2-(Meth)-acrylolyloxy-1,3-propylen, 3-(Meth)-acrylolyloxy-1,2-propylen, 2-(Meth)-acrylolyloxymethyl-2-ethyl-1,3-propylen, 2,2-Bis-(meth)-acrylolyloxymethyl-1,3-propylen.

Bevorzugt werden (Meth)-acrylsäure-Derivate von Tricyclodecanen nach Formel I, bei denen
$R^1$ und $R^2$ Wasserstoff bedeuten,
$R^3$ und $R^4$ gleich oder verschieden sind und für die Gruppe

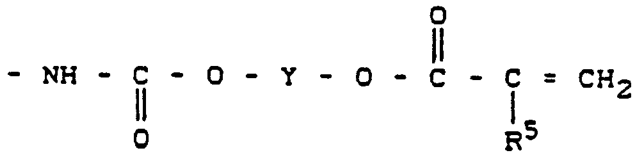

stehen, wobei

Y eine geradkettige oder verzweigte Kohlenwasserstoffkette mit 2 bis 10 Kohlenstoffatomen ist, die gegebenenfalls 1 bis 3 Sauerstoffbrücken enthalten und gegebenenfalls durch 1 bis 4 Acrylat- oder Methacrylatreste substituiert sein kann, wobei

$R^5$ für Wasserstoff oder Methyl steht.

Bei den Substituenten $R^3$ und $R^4$ handelt es sich um Methacrylatgruppen enthaltende Alkyloxycarbonylaminoreste, die man z.B. durch Umsetzung entsprechender Isocyanatgruppen mit Methacrylatgruppen enthaltenden Hydroxylverbindungen erhalten kann. Bevorzugt sind z.B. Hydroxyethyl(meth)acrylate, 2-Hydroxypropyl(meth)acrylate, Trimethylolpropandi(meth)acrylate, Propantrioldi(meth)acrylate sowie Pentaerythrittri(meth)acrylate, Dipentaerythritpenta(meth)acrylate. Auch Hydroxyverbindungen, die sowohl Acrylatals auch Methyacrylatgruppen enthalten, sind gut geeignet. Besonders bevorzugte Hydroxylverbindungen sind 2-Hydroxy-propylmethacrylat und Propandiolmethacrylat (Gemisch von 1,2- und 1,3-Methacrylat) und Hydroxy-methacrylolyloxyacrylolyloxypropan (Gemisch von 1,2- und 1,3-Diester).

Beispielsweise seien die folgenden (Meth)-acrylsäurederivate von Tricyclodecanen (Ia) genannt,

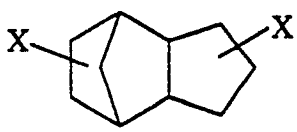

(Ia)

in denen X für folgende Substituenten steht:

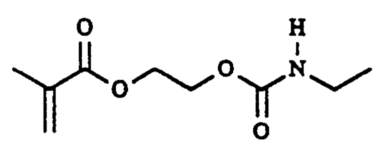

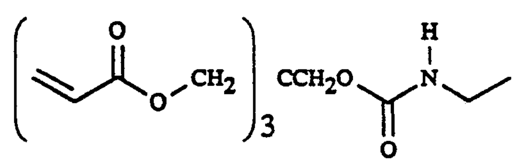

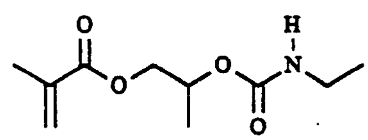

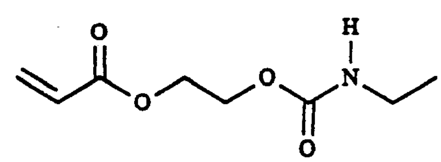

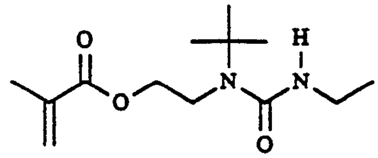

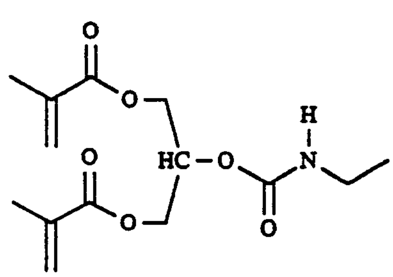

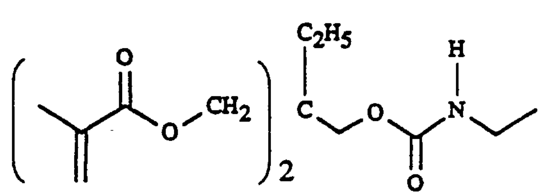

Es wurde auch ein Verfahren zur Herstellung der erfindungsgemäßen (Meth)-acrylsäure-Derivaten von tricyclodecanen gefunden, das dadurch gekennzeichnet ist, daß man Diisocyanatomethyltricyclodecane der Formel

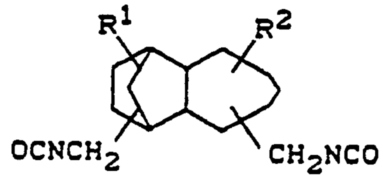

(II),

in der

$R^1$ und $R^2$ gleich oder verschieden sind und Wasserstoff, Niederalkyl, Niederalkoxy, Halogen oder Trifluoromethyl bedeuten, mit (Meth)-acrylsäure-Derivaten der Formeln

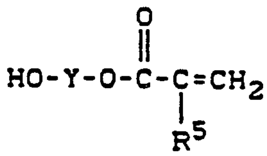

oder

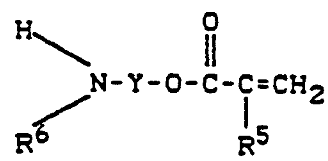

(III) (IV)

in denen

Y eine geradkettige oder verzweigte Kohlenwasserstoffkette mit 2 bis 10 Kohlenstoffatomen ist, die gegebenenfalls Saurestoffbrücken enthalten kann und durch gegebenenfalls 1 bis 4 Acrylat- oder Methacrylatreste substituiert ist, wobei

$R^5$ für Wasserstoff oder Methyl steht, gegebenenfalls in einem inerten Lösungsmittel in Gegenwart eines Katalysators im Temperaturbereich von 0 bis 100°C umsetzt.

Diisocyanatomethyl-tricyclodecane sind an sich bekannt und können beispielsweisedurch Umsetzung von Diaminomethyl-tricyclodecan mit Phosgen hergestellt werden.

(Meth)-acrylsäure-Derivate der Formel (III) sind an sich bekannt (DE 3 234 045) und können beispielsweise durch Umsetzung von Epoxiden mit (Meth)acrylsäure hergestellt werden.

(Meth)-acrylsäure-Derivate der Formel (IV) sind an sich bekannt (DAS 1 235 896) und können beispielsweise durch Umsetzung von Alkanolaminen mit Säurehalogeniden hergestellt werden.

Es ist möglich, das erfindungsgemäße Verfahren ohne Lösungsmittel druchzuführen. Inerte Lösungsmittel für das erfindungsgemäße Verfahren sind bevorzugt unpolare Lösungsmittel, die sich unter den Reaktionsbedingungen nicht verändern. Als Lösungsmittel werden insbesondere bevorzugt: Chloroform, Tetrahydrofuran, Dioxan, Methylenchlorid, Toluol, Acetonitril und Freon. Insbesondere bevorzugt werden Chloroform, Tetrahydrofuran, Freon und Acetonitril.

In einer besonderen Ausführungsform wird das erfindungsgemäße Verfahren unter Ausschluß von Wasser durchgeführt. Besonders bevorzugt wird eine maximale Menge von Wasser Unter 0,1%.

Katalysatoren für das erfindungsgemäß Verfahren sind im allgemeinen Metallsalze höherer Fettsäuren. Bevorzugte Katalysatoren sind beispielsweise Dibutylzinnlaurat oder Zinn-(II)-octoat. Bevorzugt sind aber auch Verbindungen mit tertiären Aminogruppen, wie Pyridin, Methylpyridin, N,N'-Dimethylpiperazin, N,N-Dimethylbenzylamin, u.a. und Titanverbindungen.

Im allgemeinen wird der Katalysator in einer Menge von 0,1 bis 2,5 Gew-%, bevorzugt von 0,1 bis 1,5 Gew.-%, bezogen auf die Gesamtmenge der Reaktanten eingestezt.

In einer bevorzugten Ausführungsform kann das erfindungsgemäße Verfahren in Gegenwart eines Polymerisationsinhibitors durchgeführt werden. Polymerisationsinhibitoren können beispielsweise für diesen Zweck aus (Ullmanns Encyklopädie der technischen Chemie, 4, Aufl., Verlag Chemie, Weinheim, Bd. 8, S.19—45) bekannte Verbindungen sein. Beispielsweise sie 2,6-Di-tert.-butyl-4-methylphenol genannt. Bevorzugter Polymerisationsinhibitoren ist beispielsweise auch der Sauerstoff der Luft, die in das Reaktionsgemisch eingeleitet wird.

Im allgemeinen wird der Polymerisationsinhibitor in einer Menge von 0,01 bis 0,2 Gew.-%, bevorzugt von 0,1 bis 0,05 Gew.-%, eingesetzt.

Das erfindungsgemäße Verfahren wird im allgemeinen im Temperaturebereich von 0 bis 100°C, vorzugsweise von 30 bis 70°C, durchgeführt. Das erfindungsgemäße Verfahren wird im allgemeinen bei Normaldruck durchgeführt. Est ist aber auch möglich, das erfindungsgemäße Verfahren beim Unter- oder Überdruck (beispielsweise im Druckbereich von 0,1 bis 10 bar) durchzuführen.

Das erfindungsgemäße Verfahren kann beispielsweise wie folgt durchgeführt werden:

Die Reaktanten werden in dem Lösungsmittel gelöst und unter Rühren mit dem Katalysator und gegebenenfalls dem Polymerisationsinhibitor versetzt. Der zeitliche Verlauf er Umsetzung kann beispielsweise durch Messung der IR-Spektren verfolgt werden. Nach vollständiger Umsetzung der Isocyanatgruppen werden die Reaktionsprodukte durch Entfernen des Lösungsmittels isoliert. Eine vorherige Reinigung mit Hilfe von Adsorbentien, bzw. Aktivkohle, Bleicherde, Kieselgel oder Aluminiumoxide ist möglich.

Die erfindungsgemäßen (Meth)-acrylsäure-Derivate von Tricyclodecanen können als Monomere tur Dentalmaterialien verwendet werden. Es ist möglich, sie als Monomere für polymere Zahnfüllmassen oder Beschichtungsmittel (Zahnlacke) in Dentalwerkstoffen einszusetzen.

Für die Anwendung als Monomere für polymere Zahnfüllmassen oder Beschichtungsmittel in Dentalwerkstoffen können die erfindungsgemäßen (Meth)-acrylsäure-Derivate von Tricyclodecanen mit an sich bekannten Monomeren gemischt werden, um beispielsweise die Viskosität dem Verwendungszweck anzupassen. Hierbei werden Viskositäten im Bereich von 60 bis 10,000 mPas bevorzugt. Dieses ist dadurch erreichbar, daß man den erfindungsgemäßen Monomeren gegebenenfalls ein Comonomer niedriger Viskosität als Reaktivverdünner zumischt. Die erfindungsgemäßen Verbindungen werden in der Mischung mit Comonomeren mit inem Anteil von ca. 30 bis ca. 90 Gew.-%, bevorzugt von 50 bis 80 Gew.-%, eingestzt. Zweck der vorliegenden Erfindung ist es, ebenfalls bevorzugt Mischungen verschiedener erfindungsgemäßer (Meth)-acrylsäure einzusetzen.

Es ist auch möglich, Monomermischungen einzusetzen, die mehrere Comonomere als Reaktivverdünner enthalten.

Beispielsweise seien die folgenden Comonomeren genannt: Glycerindimethacrylat, Triethylenglykoldimethacrylat, Tetraethylenglykoldimethacrylat, 1,12-Dodecandioldimethacrylat, 1,6-Hexandioldimethyacrylat, Diethylenglykoldimethacrylat, 2,2-Bis-[p-(2'-hydroxy-3'-methacryloyloxypropoxy)-phenyl]-propan, 2,2-Bis-[p-(2'-methacrylolyloxyethoxy)-phenyl]-propan. Trimethylol-propan-tri-(meth)-acrylat, Bis-(meth)-acryloyloxyethoxymethyl-tricyclo-[5.2.1.0$^{2,6}$]-decan (DE—OS 29 31 925 und DE—OS 29 31 926).

Insbesondere bevorzugt werden Comonomere, die bei 13 mbar einen Siedepunkt über 100°C besitzen.

Die erfindungsgemäßen (Meth)-acrylsäureester lassen sich gegebenenfalls in Mischungen mit den genannten Comonomeren mit an sich bekannten Methoden zu vernetzenden Polymerisaten aushärten (Am. Chem. Soc., Symp. Ser. *212*, 359—371 (1983)). Für die sogenannte Redoxypolymerisation ist ein System aus einer peroxidischen Verbindung und einem Reduktionsmittel, beispielsweise auf Basis tertiärer aromatischer Amine, geeignet. Beispiele für Peroxide sind: Dibenzoylperoxid, Dilauroylperoxid und Di-4-chlorbenzoylperoxid.

Als tertiäre aromatische Amine seien beispielsweise N,N-Dimethyl-p-toluidin, Bis-(2-hydroxyethyl)-p-toluidin, Bis-(2-hydroxyethyl)-3,5-dimethylanilin und N-Methyl-N-(2-methylcarbamoyloxypropyl)-3,5-dimethylanilin genannt. Die Konzentration des Peroxids bzw. des Amins werden vorteilhaft so gewählt, daß sie 0,1 bis 5 Gew.-%, bevorzugt 0,5 bis 3 Gew.-%, bezogen auf die Monomermischung betragen. Die peroxid- bzw. aminhaltigen Monomerischungen werden bis zur Anwendung getrennt gelagert.

Die erfindungsgemäßen Monomeren können auch durch Bestrahlung mit UV-Licht oder sichtbarem Licht (beispielsweise im Wellenlängenbereich von 230 bis 650 nm) zur Polymerisation gebracht werden. Als Initiatoren für die photoinitiierte Polymerisation eignen sich beispielsweise Benzol, Benzoldimethylketal, Benzoinmonoalkylether, Benzophenon, p-Methoxybenzophenon, Fluorenon, Thioxanthon, Phenanthrenchinon und 2,3-Bornandion (Campherchinon), gegebenenfalls Gegenwart von synergistisch wirkenden Aktivatoren, wie N,N-Dimethylaminoethylmethacrylat, Triethanolamin, 4-N,N-Dimethylaminobenzolsulfonsäurediallylamid. Die Durchführung der Photopolymerisation ist beispielsweise in der DE—PS 3 135 115 beschrieben.

Neben den oben beschriebenen Initiatoren können den erfindungsgemäßen (Meth)-acrylsäure-Derivaten an sich für diesen Einsatzzweck bekannte Lichtschutzmittel und Stabilisatoren zugesetzt werden.

Lichtschutzmittel sind beispielsweise in (Gächter, Müller, Taschenbuch der Kunstoff-Additive, 2. Ausgabe, Carl Hauser Verlag) beschrieben. Beispielsweise seien die folgenden Lichtschutzmittel genannt: Cyasorb UV9®, Tinuvin P®, Tinuvin 770®, Tinuvin 622®, Tinuvin 765®.

Stabilisatoren sind beispielsweise in (Ullmanns Encyklopädie der technischen Chemie, 4. Aufl., Bd. 8) beschrieben. Beispielsweise seien die folgenden Stabilisatoren genannt: 2,6-Di-tert.-butylphenol, 2,6-Di-tert.-butyl-4-methylphenol, 2,6-Di-octadecyl-4-methyl-phenol, 1,1'-Methylen-bis(naphthol-2) u.a.

Die Lichtschutzmittel und die Stabilisatoren können jeweils in einer Menge von 0,01 bis 0,5 Gew.-Tln, bezogen auf 100 Gew.-Tle der Monomermischung, eingesetzt werden. Die Monomermischungen können ohne Zusatz von Füllstoffen als Beschichtungsmittel (Zahnlacke) eingestzt werden.

Bei der Verwendung als Zahnfüllmassen setzt man den erhaltenen Monomermischungen im allgemeinen Füllstoffe zu. Um einen hohen Füllgrad errreichen zu können, sind Monomermischungen, die eine hohe Viskosität im Bereich von 60 bis 10.000 mPas besitzen, besonders vorteilhaft.

Vorzugsweise mischt man den erfindungsgemäßen (Meth)-acrylsäure-Derivaten von Tricyclodecanen anorganische Füllstoffe zu. Beispielsweise seien Bergkristall, Graphit, Kristoballit, Quarzglas, hochdisperse Kieselsäure, Aluminiumoxide und Glaskeramiken, beispielsweise Lantan und Zirkon enthaltende Glaskeramiken (DE—OS 23 740 501) genannt. Die anorganischen Füllstoffe werden zur Verbesserung des Verbunds zur Polymermatrix des Polymethacryu Polymermatrix des Polymethacrylats, vorzugsweise mit einem Haftvermittler vorbehandelt. Die Haftvermittlung kann beispielsweise durch eine Behandlung mit Organosiliciumverbundungen erreicht werden (Progress in Organic Coatings *11* 297—308 (1983)). Bevorzugt wird 3-Methacryloyloxipropyl -trimethoxisilan engesetzt. Die Füllstoffe für die erfindungsgemäßen Zahnfüllmassen weisen im allgemeinen einen mittleren Teilchendurchmesser von 0,01 bis 100 σm, vorzugsweise von 0,05 bis 50 σm, besonders bevorzugt von 0,05 bis 5 σm auf. Es kann auch vorteilhaft sein, mehrere Füllstoffe nebeneinander einzusetzen, die einen voneinander verschiedenen

Teilchendurchmesser besitzen.

Der Füllstoffanteil in der Zahnfüllmasse beträgt im allgemeinen 5 bis 85 Gew.-%, vorzugsweise 50 bis 80 Gew.-%.

Für die Herstellung der Zahnfüllmassen werden die Komponenten unter Verwendung handelsüblicher Knetmaschinen verwendet.

Der Anteil der erfindungsgemäßen (Meth)-acrylsäure-Derivate von Tricyclodecanen in den Füllmassen beträgt im allgemeinen 5 bis 85 Gew.-%, bezogen auf die Füllmasse.

Die erfindungsgemäßen Zahnlacke und Zahnfüllmasse haben überraschenerweise einen besonders niedrigen Polymerisationsschrumpf und gute mechanische Fertigkeiten.

Herstellungsbeispiele

1. Umestzung von 3(4), 8(9) Diisocyanatomethyl-tricyclo-(5.2.1.$0^{2,6}$)-decan mit Hydroxyethylmethacrylat.

In einem geeignet ausgerüsteten Reaktionsgefäß werden unter Rühren und Durchblasen von trockener Luft 0,8 Mol Hydroxyethylmethacrylat, 0,4 Mol Diisocyanat, 0,13 g 2,6-Di-tert.-butyl-4-methyl-phenol (Jonol), und 0,5 g Dibutylzinndilaurat 6 h bei 50°C zur Reaktion gebracht.

| | | |
|---|---|---|
| Gefunden: | % C 61,8 | Berechnet: % C 61,7 |
| | % H 7,2 | % H 7,5 |
| | % N 5,4 | % N 5,5 |

Molekulargewicht (osmometrisch): 500—511 (berechnet 506)

2. Umsetzung von 3(4), 8(9) Diisocyanatomethyl-tricyclo-(5.2.1.$0^{2,6}$)-decan mit Hydroxypropylmethacrylat.

0,8 Mol 2-Hydroxymethyacrylsäurepropylester, 0,4 Mol Diisocyanat, 0,13 g Jonol und 0,15 g Dibutylzinndilaurat werden wie in I.) beschrieben zur Reaktion gebracht.

| | | |
|---|---|---|
| Gefunden: | % C 62,6 | Berechnet: % C 62,9 |
| | % H 5,2 | % H 5,2 |
| | % N 8,1 | % N 7,9 |

Molekulargewicht (osmometrisch): 527 (berechnet 534) Viskosität (25°C): 356.000 mPas.

3. Umsetzung von 3(4), 8(9) Diisocyanatomethyl-tricyclo-(5.2.1.$0^{2,6}$)-decan mit Hydroxypropylmethacrylat.

In einem geeignet ausgerüsteten Reaktionsgefäß werden 0,7 Mol N-t-butylaminoethylmethacrylat vorgelegt und unter Rühren und Durchleiten von trockener Luft langsam 0,35 Mol Diisocyanat zugegeben. Nach Beendigung der Zugabe wird die Reaktionsmischung noch 30 min. nachgerührt.

4. Umsetzung von 3(4), 8(9) Diisocyanatomethyl-tricyclo-(5.2.1.$0^{2,6}$)-decan mit Glycerindimethacrylat.

In einem geeignet ausgerüsteten Reaktionsgefäß werden 114 g Glycerindimethacrylat 0,5 g Dibutylzinndilaurat und 0,063 g Jonol in 200 ml Methylenchlorid gelöst. In diese Lösung tropft man bei Raumtemperatur 615 g Diisocyanat. Anschließend wird das Reaktionsgemisch 50 Stunden bei 40—50°C gerührt. Nach Abdestillieren des Lösungsmittels am Rotationsverdampfer erhält man eine klare, farblose Flüssigkeit.

IR ($cm^{-1}$): 3380 (N—H); 1725 (C=O) 1715 (C=O); 1160 (C—O)

$^1$H—NMR (ppm) in $CDCl_3$/TMS: 6.16, 5.62 (=$CH_2$, 8H); 5.1—5.5 (=$CH_3$—4H); 4.2—4.5 (COO—$CH_2$—, 8H), 3.0 (—CONH—$CH_2$—, 4H), 1.95, 2.5—2.0, 1.7—1 ($CH_3$, TCD—$CH_2$, 26H).

| | | |
|---|---|---|
| Gefunden: | % C 66,3 | Berechnet: % C 66,2 |
| | % H 8,6 | % H 9,1 |
| | % N 9,0 | % N 9,1 |

Molekulargewicht (osmometrisch): 627 (berechnet 616) Viskosität (25°C): 1.220.000 mPas.

Anwendungsbeispiele

5. Messung des Polymerisationsschrumpfes:

Im reinen Monomer werden 2% Benzoylperoxid gelöst. 5 g dieser Lösung werden in ein zylindrisches Glasgefäß von 3 cm Durchmesser gefüllt und mit Stickstoff überschichtet. Die Lösungen werden 1 h auf 80°C und 15 min. auf 130°C erhitzt, wobei die Monomeren polymerisieren. Von den erhaltenen

Probekörpern wird die Dichte bestimmt und durch Vergleich mit der Dichte der flüssigen Monomeren der Polymerisationsschrumpf bestimmt.

Tabelle I

Vergleichsversuche Monomer — Polymerisationsschrumpf

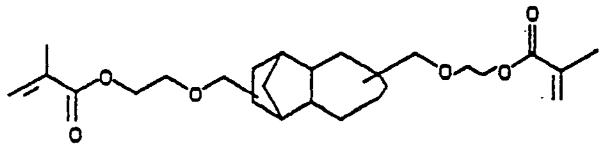

7.3 %

(DE-OS 29 31 926)

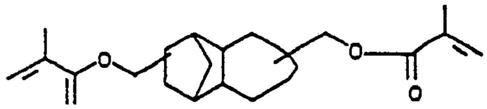

7.7 %

(DE-OS 28 16 823)

Erfindungsgemäße Monomere

6.0 %

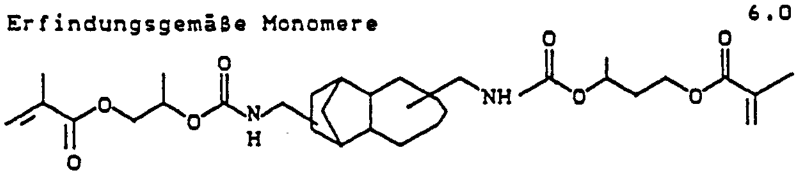

6.3 %

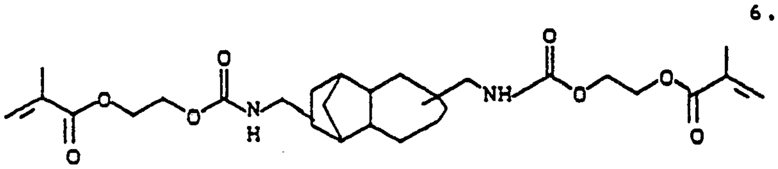

6. Masse zum Füllen von Zahnhohlräumen:

a) Redoxhärtendes System

Peroxidpaste: In einer Mischung aus 70 Teilen Monomer aus 1.) und 30 Teilen Triethylenglycoldimethacrylat werden 2% Benzoylperoxid gelöst. 10 g silanisierte Glaskeramik werden mit 4 g dieser Lösung zu einer Paser verarbeitet.

Aminpaste: In einer Mischung von 70 Teilen 1.a) und 30 Teilen Triethylenglycoldimethacrylat werden 1,3% N-Methyl-N-β-(methylcarbamoyloxy)-propyl-3,5-dimethylanilin gelöst. 4 g dieser Lösung werden mit 10 g silanisierter Glaskeramik zu einer Paste verarbeitet.

Werden gleiche Teile Aminpaste und Peroxidpaste miteinander gemischt, so härtet die Mischung in 2 Minuten aus. Die Pasten können mit Pigmenten eingefärbt werden und eignen sich zum Füllen von Zahnhohlräumen.

b) Lichthärtendes System

In einer Mischung von 700 Teilen Monomer aus 1.) und 30 Teilen Triethylenglycoldimethacrylat werden 0,5% N,N-Diallyly-p-dimethylaminobenzolsulfonsäureamid, 0,2% Campherchinon und 0,125% Benzildimethylketal gelöst, 10 g silanisierte Glaskeramik werden mit 4 g dieser Lösung zu einer Paste verarbeitet. Bestrahlt man diese Masse mit einer handelsüblichen Dentallampe (Translux, Fa. Kulzer), so ist nach 40 sec eine Schicht von 7 mm durchgehärtet.

7. Herstellung von Sealerlösungen:

a) Redoxhärtendes system

Katalysatorlösung: In einer Mischung aus 10 Teilen Triethylenglycoldimethacrylat und 90 Teilen Monomer 2) werden 2% Benzoylperoxid gelöst.

Aktivatorlösung: In einer Mischung aus 10 Teilen Triethylenglycoldimethacrylat und 90 Teilen Monomer 2) werden 2,15% N-Methyl-N-β-(methylcarbamoyloxy)-propyl-3,5-dimethylanilin gelöst.

Eine Mischung aus gleichen Teilen Katalysatorlösung und Aktivatorlösung härtet in 1 min aus.

b) Lichthärtender Sealer

In einer Mischung aus 30 Teilen Triethylenglycoldimethacrylat und 70 Teilen Monomer 3) werden 0,5% N,N-Diallyl-p-dimethylaminobenzolsulfonsäureamid, 0,2% Campherchinon und 0,125% Benzildimethylketal gelöst.

Beim Bestrahlen mit einer handelsüblichen Dentallampe (Translux, Fa. Kulzer) härtet die Flüsigkeit zu einem festen Film aus.

**Patentansprüche**

1. (Meth)-acrylsäure-Derivate von Tricyclodecanen der Formel

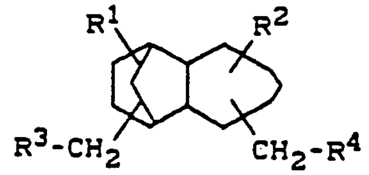

,

in der

$R^1$ und $R^2$ gleich oder verschieden sind und Wasserstoff, Niederalkyl, Niederalkoxy, Halogen oder Trifluormethyl bedeuten, und

$R^3$ und $R^4$ gleich oder verschieden sind und für die Gruppen

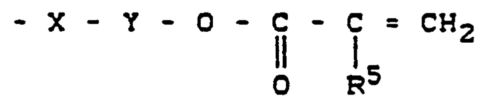

stehen, wobei

X ein zweibindiges Brückenglied aus der Gruppe

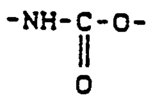

oder

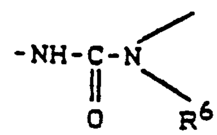

und

Y eine geradkettige oder verzweigte Kohlenwasserstoffkette mit 2 bis 10 Kophlenstoffatomen ist, die gegebenenfalls Saurestoffbrücken enthalten kann und durch gegebenenfalls 1 bis 4 Acrylat- oder Methacrylatreste substituiert ist, wobei

$R^5$ für Wasserstoff oder Methyl und

$R^6$ für Niederalkyl oder Phenyl stehen.

2. (Meth)-acrylsäure-Derivate von Tricyclodecanen nach Anspruch 1, wobei

$R^1$ und $R^2$ Wasserstoff bedeuten,

$R^3$ und $R^4$ gleich oder verschieden sind und für die Gruppe

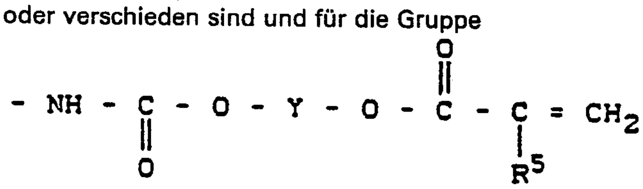

stehen, wobei

Y eine geradkettige oder verzweigte Kohlenwasserstoffkette mit 2 bis 10 Kohlenstoffatomen ist, die gegebenenfalls 1 bis 3 Sauerstoffbrücken enthalten und gegebenenfalls durch 1 bis 4 Acrylat- oder Methacrylatreste substituiert sein kann, wobei

$R^5$ für Wasserstoff oder Methyl steht.

3. Verfahren zur Herstellung von (Meth)-acrylsäure-Derivate von Tricyclodecanen, dadruch gekennzeichnet, daß man Diisocyanatomethyltricyclodecane der Formel

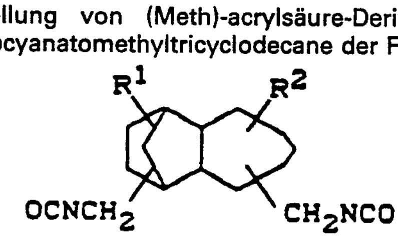

in der

$R^1$ und $R^2$ gleich oder verschieden sind und Wasserstoff, Niederalkyl, Niederalkoxy, Halogen oder Trifluormethyl bedeuten, mit (Meth)-acrylsäure-Derivaten der Formeln

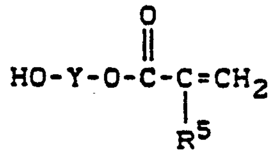

oder

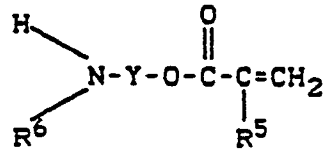

in denen

Y eine geradkettige oder verzweigte Kohlenwasserstoffkette mit 2 bis 10 Kohlenstoffatomen ist, die

gegebenenfalls Saurestoffbrücken enthalten kann und durch gegebenenfalls 1 bis 4 Acrylat- oder Methacrylatreste substituiert ist, wobei

$R^5$ für Wasserstoff oder Methyl steht, gegebenenfalls in einem inerten Lösungsmittel in Gegenwart eines Katalysators im Temperaturbereich von 0 bis 100°C umsetzt.

4. Polymerisat aus (Meth)-acrylsäure-Derivaten von Tricyclodecanen der Formel

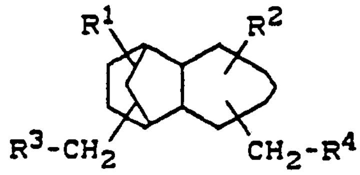,

in der

$R^1$ und $R^2$ gleich oder verschieden sind und Wasserstoff, Niederalkyl, Niederalkoxy, Halogen oder Trifluormethyl bedeuten, und

$R^3$ und $R^4$ gleich oder verschieden sind und für die Gruppen

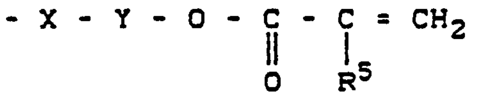

stehen, wobei

X Sauerstoff oder ein zweibindiges Brückenglied aus der Gruppe

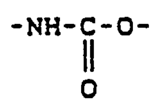 oder 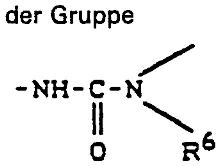

und

Y eine geradkettige oder verzweigte Kohlenwasserstoffkette mit 2 bis 10 Kohlenstoffatomen ist, die gegebenenfalls Saurestoffbrücken enthalten kann und durch gegebenenfalls 1 bis 4 Acrylat- oder Methacrylatreste substituiert ist, wobei

$R^5$ für Wasserstoff oder Methyl und

$R^6$ für Niederalkyl oder Phenyl stehen.

5. Verwendung von (Meth)-acrylsäure-Derivaten von Tricyclodecanen nach Anspruch 1 in Dentalwerkstoffen.

6. Verwendung nach Anspruch 5, dadurch gekennziechnet, daß die (Meth)-acrylsäure-Derivate von Tricyclodecanen in Zahnfüllmassen eingestzt werden.

7. Verwendung nach Anspruch 6, dadurch gekennzeichnet, daß die (Meth)-acrylsäure-Derivate von Tricyclodecanen in Beschichtungsmittel für Zähne eingesetzt werden können.

8. Zahnfüllmassen, dadurch gekennzeichnet, daß sie die (Meth)-acrylsäure-Derivate von Tricyclodecanen der Formel

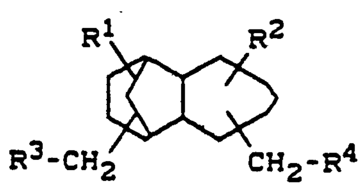,

in der

$R^1$ und $R^2$ gleich oder verschieden sind und Wasserstoff, Niederalkyl, Niederalkoxy, Halogen oder Trifluormethyl bedeuten, und

$R^3$ und $R^4$ gleich oder verschieden sind und für die Gruppen

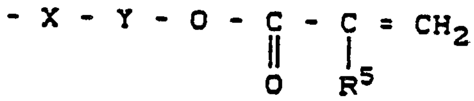

stehen, wobei

X ein zweibindiges Brückenglied aus der Gruppe

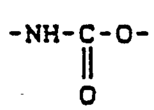 oder 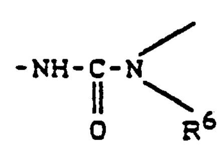

und

Y eine geradkettige oder verzweigte Kohlenwasserstoffkette mit 2 bis 10 Kohlenstoffatomen ist, die gegebenenfalls Saurestoffbrücken enthalten kann und durch gegebenenfalls 1 bis 4 Acrylat- oder Methacrylatreste substituiert ist, wobei

$R^5$ für Wasserstoff oder Methyl und

$R^6$ für Niederalkyl oder Phenyl stehen, enthalten.

9. Zahnfüllmassen nach Anspruch 8, dadurch gekennzeichnet, daß sie neben (Meth)-acrylsäure-Derivat von Tricyclodecanen ein weiteres Comonomer enthalten.

## Revendications

1. Dérivés (méth)-acryliques de tricyclodécanes, répondant à la formule

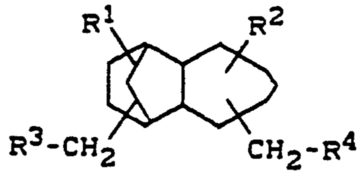

,

dans laquelle

$R^1$ et $R^2$ sont identiques ou différentes et représentent un atome d'hydrogène, un groupe alkyle inférieur, un groupe alcoxy inférieur, un atome d'halogène ou un groupe trifluorométhyle, et

$R^3$ et $R^4$ sont identiques ou différents et représentent les groupes

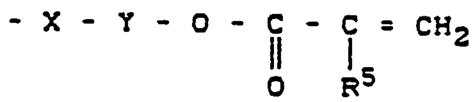

où

X représente un pont à deux liaisons choisi dans le groupe

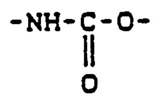 ou 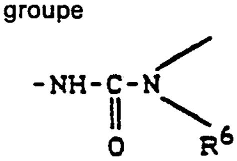

et

Y représente une chaîne hydrocarbonée droite ou ramifiée contenant 2 à 10 atomes de carbone, qui peut éventuellement contenir des ponts d'oxygène et qui est substituée par éventuellement 1 à 4 radicaux acrylate ou méthacrylate, où

$R^5$ représente un atome d'hydrogène ou un groupe méthyle et

$R^6$ représente un groupe alkyle inférieur ou un groupe phényle.

2. Dérivés (méth)-acryliques de tricyclodécanes, selon la revendication 1, dans lesquels

$R^1$ et $R^2$ représent un atome d'hydrogène,

$R^3$ et $R^4$ sont identiques ou différentes et représentent le groupe

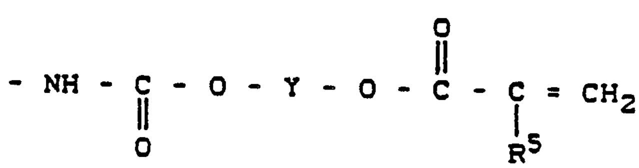

où

Y représente un chaîne hydrocarbonée droite ou ramifiée contenant 2 à 10 atomes de carbone, qui peut éventuellement contenir 1 à 3 ponts d'oxygène et éventuellement être substituée par 1 à 4 radicaux acrylate ou méthacrylate, où

$R^5$ représente un atome d'hydrogène ou un groupe méthyle.

3. Procédé de préparation de dérivés (méth)-acryliques de tricyclodécanes, caractérisé en ce que l'on fiat réagir des diisocyanatométhyl-tricyclodécanes répondant à la formule

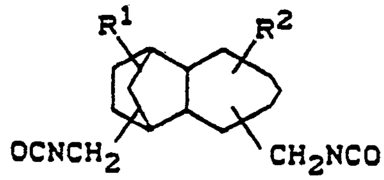

dans laquelle

$R^1$ et $R^2$ sont identiques ou différentes et représentent un atome d'hydrogène, un groupe alkyle inférieur, un groupe alcoxy inférieur, un atome d'halogène ou un groupe trifluorométhyle, avec des dérivés (méth)-acryliques répondant aux formules

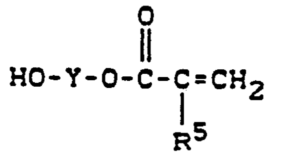

ou

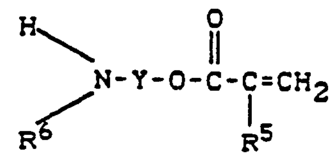

dans lesquelles

Y représente un chaîne hydrocarbonée droite ou ramifiée contenant 2 à 10 atomes de carbone, qui peut éventuellement contenir des ponts d'oxygène et qui est substituée par éventuellement 1 à 4 radicaux acrylate ou méthacrylate, où

$R^5$ représente un atome d'hydrogène ou un groupe méthyle, éventuellement dans un solvant inerte, en présence d'un catalyseur, dans un intervalle de température allant de 0 à 100°C.

4. Polymérisat constitué de dérivés (méth)-acryliques de tricyclodécanes, répondant à la formule

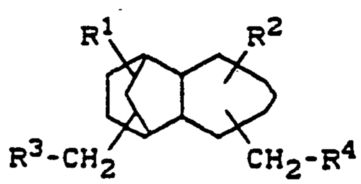

,

dans laquelle

$R^1$ et $R^2$ sont identiques ou différentes et représentent un atome d'hydrogène, un groupe alkyle inférieur, un groupe alcoxy inférieur, un atome d'halogène ou un groupe trifluorométhyle, et

$R^3$ et $R^4$ sont identiques ou différentes et représentent les groupes

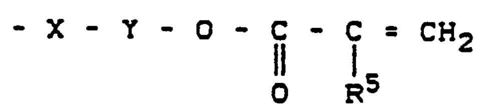

où

X représente un atome d'oxygène ou un pont à deux liaisons choisi dans le groupe

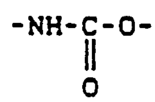

ou

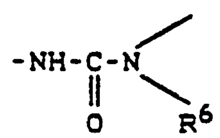

et

Y représente un chaîne hydrocarbonée droite ou ramifiée contenant 2 à 10 atomes de carbone, qui peut éventuellement contenir des ponts d'oxygène et qui est substituée par éventuellement 1 à 4 radicaux acrylate ou méthacrylate, où

$R^5$ représente un atome d'hydrogène ou un groupe méthyle et

$R^6$ représente un groupe alkyle inférieur ou un groupe phényle.

5. Utilisation de dérivés (méth)-acryliques de tricyclodécanes selon la revendication 1, dans des matériaux dentaires.

6. Utilisation selon la revendication 5, caractérisée en ce qu'on incorpore les dérivés (méth)-acryliques de tricyclodécanes dans des matières d'obturation dentaire.

7. Utilisation selon la revendication 6, caractérisée en ce qu'on incorpore les dérivés (méth)-acryliques de tricyclodécanes dans des agents d'enduction pour les dents.

8. Matières d'obturation dentaire, caractérisées en ce qu'elles contiennent les dérivés (méth)-acryliques de tricyclodécanes, répondant à la formule

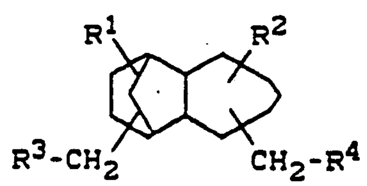

,

dans laquelle

$R^1$ et $R^2$ sont identiques ou différentes et représentent un atome d'hydrogène, un groupe alkyle inférieur, un groupe alcoxy inférieur, un atome d'halogène ou un groupe trifluorométhyle, et

$R^3$ et $R^4$ sont identiques ou différentes et représentent les groupes

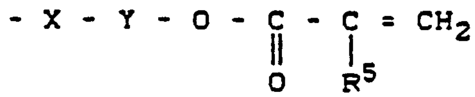

où

X représente un pont à deux liaisons choisi dans le groupe

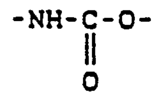

or

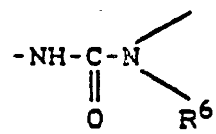

et

Y représente un chaîne hydrocarbonée droite ou ramifiée contenant 2 à 10 atomes de carbone, qui peut éventuellement contenir des ponts d'oxygène et qui est substituée par éventuellement 1 à 4 radicaux acrylate ou méthacrylate, où

$R^5$ représente un atome d'hydrogène ou un groupe méthyle et

$R^6$ représente un groupe alkyle inférieur ou un groupe phényle.

9. Matières d'obturation dentaire selon la revendication 8, caractérisées en ce qu'elles contiennent un autre comonomère, en plus du dérivé (méth)-acrylique de tricyclodécanes.

**Claims**

1. (Meth)-acrylic acid derivatives of tricyclodecanes of the formula

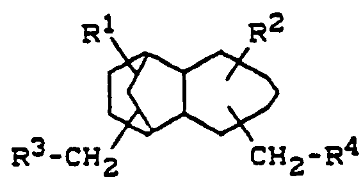

,

in which

$R^1$ and $R^2$ are identical or different and denote hydrogen, lower alkyl, lower alkoxy, halogen or trifluoromethyl and

$R^3$ and $R^4$ are identical or different and represent the groups

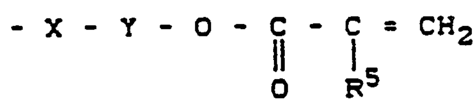

in which

X is a divalent bridge member from the group comprising

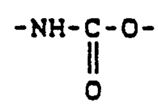

or

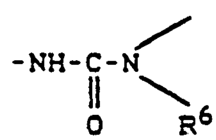

and

Y is a linear or branched hydrocarbon chain which has 2 to 10 carbon atoms and can optionally contain oxygen bridges and is optionally substituted by 1 to 4 acrylate or methacrylate radicals, and

$R^5$ represents hydrogen or methyl and

$R^6$ represents lower alkyl or phenyl.

2. (Meth)-acrylic acid derivatives of tricyclodecanes according to Claim 1, wherein

$R^1$ and $R^2$ denote hydrogen,

$R^3$ and $R^4$ are identical or different and represent the group

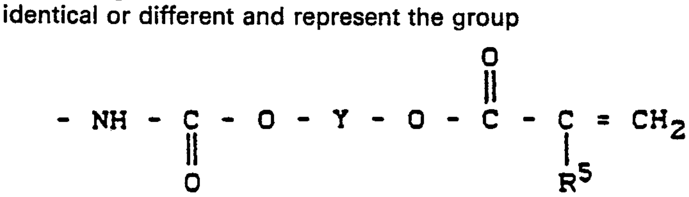

in which

Y is a linear or branched hydrocarbon chain which has 2 to 10 carbon atoms and can optionally contain 1 to 3 oxygen bridges and can optionally be substituted by 1 to 4 acrylate or methacrylate radicals, and

$R^5$ represents hydrogen or methyl.

3. Process for the preparation of (meth)-acrylic acid derivatives of tricyclodecanes, characterised in that diisocyanatomethyltricyclodecanes of the formula

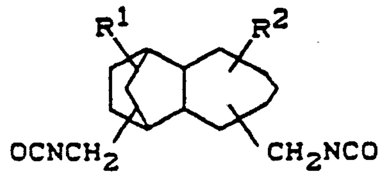

,

in which

$R^1$ and $R^2$ are identical or different and denote hydrogen, lower alkyl, lower alkoxy, halogen or trifluoromethyl, are reacted, if appropriate in an inert solvent, in the presence of a catalyst within the temperature range from 0 to 100°C, with (meth)-acrylic acid derivatives of the formulae

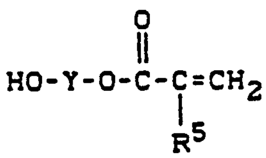

ou

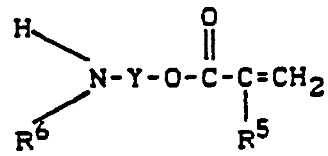

in which

Y is a linear or branched hydrocarbon chain which has 2 to 10 carbon atoms and can optionally contain oxygen bridges and is optionally substituted by 1 to 4 acrylate or methacrylate radicals, and

$R^5$ represents hydrogen or methyl.

4. Polymer formed from (meth)-acrylic acid derivatives of tricyclodecanes of the formula

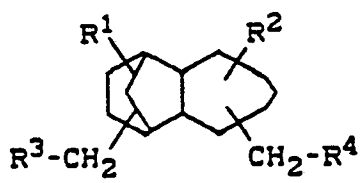

,

in which

$R^1$ and $R^2$ are identical or different and denote hydrogen, lower alkyl, lower alkoxy, halogen or trifluoromethyl and

$R^3$ and $R^4$ are identical or different and represent the groups

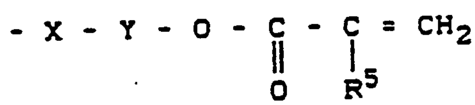

in which

X is oxygen or a divalent bridge member from the group comprising

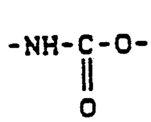

or

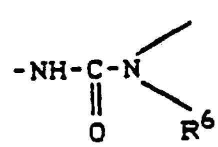

and

Y is a linear or branched hydrocarbon chain which has 2 to 10 carbon atoms and can optionally contain oxygen bridges and is optionally substituted by 1 to 4 acrylate or methacrylate radicals, and

$R^5$ represents hydrogen or methyl and

$R^6$ represents lower alkyl or phenyl.

5. The use of (meth)-acrylic acid derivatives of tricyclodecanes according to Claim 1 in dental materials.

6. Use according to Claim 5, characterised in that the (meth)-acrylic acid derivatives of tricyclodecanes are employed in tooth-filling compositions.

7. Use according to Claim 6, characterised in that the (meth)-acrylic acid derivatives of tricyclodecanes can be employed in coating agents for teeth.

8. Tooth-filling compositions characterised in that they contain (meth)-acrylic acid derivatives of tricyclodecanes of the formula

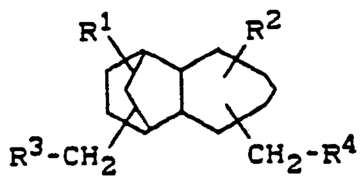 ,

in which

$R^1$ and $R^2$ are identical or different and denote hydrogen, lower alkyl, lower alkoxy, halogen or trifluoromethyl and

$R^3$ and $R^4$ are identical or different and represent the groups

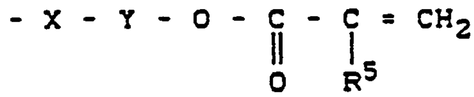

in which

X is a divalent bridge member from the group comprising

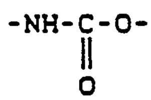 or 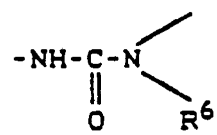

and

Y is a linear or branched hydrocarbon chain which has 2 to 10 carbon atoms and can optionally contain oxygen bridges and is optionally substituted by 1 to 4 acrylate or methacrylate radicals, and

$R^5$ represents hydrogen or methyl and

$R^6$ represents lower alkyl or phenyl.

9. Tooth-filling compositions according to Claim 8, characterised in that they contain a further comonomer in addition to a (meth)-acrylic acid derivative of tricyclodecanes.